# EUROPEAN PATENT SPECIFICATION

(11) **EP 2 124 962 B1**
(45) Date of publication and mention of the grant of the patent: **09.04.2014**
(21) Application number: 08702170.5
(22) Date of filing: 10.01.2008
(51) Int. Cl.: A61K 31/60, A61K 38/17, A61P 9/10

(54) **USE OF BIGLYCAN OR ENHANCERS OF BIGLYCAN ACTIVITY IN THE PREPARATION OF PHARMACEUTICAL COMPOSITIONS**
VERWENDUNG VON BIGLYCAN ODER MITTELN ZUR VERSTÄRKUNG DER AKTIVITÄT VON BIGLYCAN BEI DER HERSTELLUNG VON PHARMAZEUTISCHEN ZUSAMMENSETZUNGEN
UTILISATION DE BIGLYCANE OU D'ACTIVATEURS DE BIGLYCANE DANS LA PRÉPARATION DE COMPOSITIONS PHARMACEUTIQUES

(30) Priority: 11.01.2007 HU 0700024
(43) Date of publication of application: 02.12.2009
(73) Proprietor: Pharmahungary 2000 Kft., 6722 Szeged (HU)
(72) Inventor: SÁNTHA, Miklós, H-6726 Szeged (HU); GONDA, Szilvia, H-6724 Szeged (HU); BERECZKI, Erika, SE-141 86 Huddinge (SE); FERDINANDY, Péter, H-6726 Szeged (HU); CSONT, Tamás, H-6726 Szeged (HU)
(74) Representative: Török, Ferenc
(86) International application number: PCT/HU2008/000003
(87) International publication number: WO 2008/084268

(56) References cited:
- WO-A-94/20123
- GB-A- 1 206 790
- SHEHADEH ABBAS A ET AL: "Nonplatelet effects of aspirin during acute coronary occlusion: Electrophysiologic and cation alterations in ischemic myocardium" JOURNAL OF CARDIOVASCULAR PHARMACOLOGY AND THERAPEUTICS, vol. 5, no. 2, April 2000 (2000-04), pages 113-120, XP009098218 ISSN: 1074-2484
- HANSSON GORAN K ET AL: "Inflammation and atherosclerosis", ANNUAL REVIEW OF PATHOLOGY-MECHANISMS OF DISEASE, vol. 1, no. 1, 2006, pages 297-329, XP009145346, ISSN: 1553-4006
- MAURIELLO A ET AL: "Diffuse and Active Inflammation Occurs in Both Vulnerable and Stable Plaques of the Entire Coronary Tree", JOURNAL OF THE AMERICAN COLLEGE OF CARDIOLOGY, ELSEVIER, NEW YORK, NY, US, vol. 45, no. 10, 17 May 2005 (2005-05-17), pages 1585-1593, XP004889597, ISSN: 0735-1097, DOI: DOI:10.1016/J.JACC.2005.01.054

## Description

The present invention relates to the use of biglycan or enhancers of biglycan activity in the preparation of pharmaceutical compositions having anti-atherosclerotic and anti-ischemic effect, and to the use of them in methods for preventing and treating the atherosclerotic and ischemic (e.g. cardiac) diseases. The invention also relates to screening methods by which biglycan or enhancers of biglycan activity can be identified.

### BACKGROUND OF THE INVENTION:

### Biglycan and its role in the cardiovascular system

Biglycan is a member of the small leucine rich proteoglycan (SLRP) family, which is characterized by the presence of repeated leucine rich amino acid motif [Fisher, L.W., Termine, J.D., and Young, M.F.: Deduced protein sequence of bone small proteoglycan I (biglycan) shows homology with proteoglycan II (decorin) and several nonconnective tissue proteins in a variety of species. J. Biol. Chem. 264, 4571-4576 (1989)].

Recent results showed that proteoglycans are not merely rigid components of the extracellular matrix, but play an important role in deposition of collagens, activation and inactivation of cytokines and growth factors. Concerning the pharmaceutical application of biglycan it should be mentioned that US patent application No. 20050059580 discloses that biglycan can be applied in method for treating and preventing conditions associated with abnormal dystrophin-associated protein complex (DAPC), especially in muscular dystrophy.

Biglycan has been found in almost every tissue within our body, but it is not uniformly distributed within the organs. It has been shown to be expressed on the cell surface, pericellulary, and within the extracellular matrix [Bianco P., Fisher, L.W., Young, M.F., Termine, J.D., and Gehron Robey, P.: Expression and localization of the two small proteoglycans biglycan and decorin in developing human skeletal and non-skeletal tissues. J. Histochem. Cytochem. 38, 1549-1568 (1990); Wadhwa S, Embree MC, Bi Y, Young M: Regulation, regulatory activities, and function of biglycan. Crit Rev Eukaryot Gene Expr. 2004;14(4):301-315.]. Its expression pattern is altered in different pathological conditions.

Biglycan expression is regulated by transcriptional and non-transcriptional mechanisms. The function of biglycan is dependent on its microenvironment, so it depends on the tissue and pathologies studied. In the cardiovascular system, biglycan may play a crucial role in atherosclerosis [Fedarko, N.S., Termine, J.D., Young, M.F., and Robey, P.G.: Temporal regulation of hyaluronan and proteoglycan metabolism by human bone-cells invitro. J. Biol. Chem. 265, 12200-12209 (1990); Klezovitch, O. and Scanu, A.M.: Domains of Apolipoprotein E Involved in the Binding to the Protein Core of Biglycan of the Vascular Extracellular Matrix: Potential Relationship between Retention and Anti-Atherogenic Properties of this Apolipoprotein. Trends Cardiovasc. Med. 11, 263-268 (2001)]. Biglycan expression in the heart is ubiquitous, with strong filamentous strands in the central layer of the heart leaflet [Latif, N., Sarathchandra, P., Taylor, P.M., Antoniw, J., and Yacoub, M.H.: Localization and pattern of expression of extracellular matrix components in human heart valves. J. Heart Valve Dis. 14, 542-548 (2005)].

It has been demonstrated that different proteoglycan (PG) types are present in aortic layers in variable amounts where they participate in structural integrity of the aortic wall, as well as in several biological functions, such as lipoprotein oxidation and blood coagulation [Wight, T.N., Kinsella, M.G., and Qwarnstrom, E.E.: The role of proteoglycans in cell adhesion, migration and proliferation. Curr. Op. Cell Biol. 4, 793-801(1992) and Camejo, G., Hurt-Camejo, E., Wiklund, O., and Bondjers, G.: Association of apo B lipoproteins with arterial proteoglycans: Pathological significance and molecular basis. Atherosclerosis 139, 205-222 (1998)].

In arterial blood vessels, smooth muscle cells synthesize biglycan. The formation of atherosclerotic plaques in blood vessels is associated with an increase in biglycan expression [Riessen, R., Isner, J.M., Blessing, E., Loushin, C., Nikol, S., and Wight, T.N.: Regional differences in the distribution of the proteoglycans biglycan and decorin in the extracellular matrix of atherosclerotic and restenotic human coronary arteries. Am. J. Pathol. 144, 962 -974 (1994)]. TGF-β was identified as positive regulator of biglycan expression [Breuer, B., Schmidt, G., and Kresse, H.: Non-uniform influence of transforming growth factor-beta on the biosynthesis of different forms of small chondroitin sulphate/dermatan sulphate proteoglycan. Biochem. J. 269, 551-554 (1990)].

Abnormal mechanical stress in the prelesional arteries leads to increased biglycan expression via TGF-β regulation. Factors associated to atherosclerotic plaques have also been shown to cause an increase in biglycan expression [Chang, M.Y., Tsoi, C., Wight, T.N., and Chait, A.: Lysophosphatidylcholine Regulates Synthesis of Biglycan and the Proteoglycan Form of Macrophage Colony Stimulating Factor. Arterioscler Thromb Vasc Biol 23, 809-815 (2003)].

Based on the above articles (see Fedarko et al., Klezovitch et al., Riessen et al. and Chang et al.) a skilled person should draw the conclusion that an increase in the level of biglycan should facilitate the atherosclerotic plaques formation, i.e. it should exert a negative effect in this disorder.

Recently it was shown that the expression of biglycan is downregulated by the inflammatory mediator nitric oxide (NO) [Schaefer, L., Beck, K.F., Raslik, I., Walpen, S., Mihalik, D., Micegova, M., Macakova, K., Schonherr,E. and others: Biglycan, a Nitric Oxide-regulated Gene, Affects Adhesion, Growth, and Survival of Mesangial Cells. J. Biol. Chem. 278, 26227-26237 (2003)]. NO is an important cardioprotective molecule via its vasodilator, antioxidant, antiplatelet, and antineutrophil actions and it is essential for normal heart function [Ferdinandy, P. and Schulz, R.: Nitric oxide, superoxide, and peroxynitrite in myocardial ischaemiareperfusion injury and preconditioning. Br. J. Pharmacol. 138, 532-543 (2003)]. NO is produced in vivo by a group of three NO synthases (NOSs): neuronal (nNOS), inducible (iNOS), and endothelial (eNOS) isoforms. All three NOS isoforms are found in the human heart. Unlike constitutively expressed NOS isoforms (nNOS and eNOS), iNOS is regulated primarily at the transcriptional level. Normally there is little, if any, detectable iNOS expression in any cell type. However, in response to inflammatory cytokines or endotoxins there is a robust upregulation of iNOS mRNA and protein in virtually every nucleated cell type [Nathan, C.: Inducible nitric oxide synthase: What difference does it make? J. Clin. Invest 100, 2417-2423 (1997)].

Induction of iNOS expression is mediated through cytokine-inducible transcription factors, such as IFN regulatory factor-1 and NF-κB, which can directly bind to specific sequence elements within the iNOS promoter [Kamijo R, Harada H, Matsujama T, Bosland M, Gerecitano J, Shapiro D, Le J, Koh Sl and others: Requirement for transcription factor IRF-1 in NO synthase induction in macrophages. Science 263, 1612-1615 (1994); Xie, Q.W., Cho, H., Kashiwabara, Y., Baum, M., Weidner, J.R., Elliston, K., Mumford, R., and Nathan, C.: Carboxyl terminus of inducible nitric oxide synthase. Contribution to NADPH binding and enzymatic activity. J. Biol. Chem. 269, 28500-28505 (1994)].

NO production by eNOS and nNOS is pulsatile and calcium dependent (calcium/calmodulin complex is needed for NOS activation), whereas NO production by iNOS is continuous and calcium independent [Blatter, L.A., Taha, Z., Mesaros, S., Shacklock, P.S., Wier, W.G., and Malinski, T.: Simultaneous Measurements of Ca2+ and Nitric Oxide in Bradykinin-Stimulated Vascular Endothelial Cells. Circ Res 76, 922-924 (1995)]. It was recently shown, that biglycan acts as a proinflammatory protein activating TNF-α and macrophage inflammatory protein-2 (MIP-2) via p38, ERK and NF-κB in macrophages [Schaefer, L., Babelova, A., Kiss, E., Hausser, H.J., Baliova, M., Krzyzankova, M., Marsche, G., Young, M.F. and others: The matrix component biglycan is proinflammatory and signals through Toll-like receptors 4 and 2 in macrophages. J. Clin. Invest. 115, 2223-2233 (2005)].

Our aim was to study the role of biglycan in cellular signal transduction especially on NO-mediated mechanisms in blood vessels and in the heart.

It should be emphasized here, that in some other parts of the description the word "biglycan" is used in a broader sense, see in the "Definition of terms" part.

### Ischemic heart disease and cardioprotection: role of hyperlipidemia

Ischemic heart disease is the leading cause of death in the industrialized world. The treatment of this condition has entered a new era where mortality can be approximately halved by procedures which allow the rapid return of blood flow to the ischemic zone of the myocardium, i.e. reperfusion therapy.

Reperfusion, however, may lead to further complications such as diminished cardiac contractile function (stunning) and arrhythmia. Moreover, there is experimental evidence that irreversible cell injury leading to necrosis and apoptosis may be enhanced by reperfusion. Therefore, development of anti-ischemic (anti-ischemic effect is termed cardioprotective, when the anti-ischemic treatment protects the heart tissue against ischemia/reperfusion injury) agents to improve myocardial function, decrease the incidence of arrhythmias, delay the onset of necrosis and limit the total extent of infarction during ischemia/reperfusion is of great clinical importance.

Earlier pharmacological approaches to attenuate the consequences of ischemia/reperfusion injury have been of limited experimental efficacy or have failed to translate into useful clinical treatments. However, more recently the heart has been shown to possess a remarkable ability to adapt to ischemia/reperfusion stress and this molecular plasticity of the heart in ischemia/reperfusion has been the focus of intense research in the hope that the underlying mechanisms may be amenable to therapeutic exploitation. Ischemic preconditioning is a well-described adaptive response in which brief exposure to ischemia markedly enhances the ability of the heart to withstand a subsequent ischemic injury [see for a review: Przyklenk K, Kloner RA. Ischemic preconditioning: exploring the paradox. Prog. Cardiovasc. Dis. 40(6):517-47 (1998)].

Moreover, brief cycles of ischemia/reperfusion applied following a longer period of ischemia also confer cardioprotection against the consequences of myocardial ischemia/reperfusion, a phenomenon called ischemic postconditioning. The discovery of these two major forms of endogenous anti-ischemic, cardioprotective mechanisms has encouraged the exploration of new ways to protect the ischemic/reperfused myocardium and has amplified our knowledge of the molecular basis of injury and survival during ischemia/reperfusion [see for review: Baxter GF, Ferdinandy P. Delayed preconditioning of myocardium: current perspectives. Basic Res. Cardiol. 96:329-44 (2001)]. The mechanism by which the heart is able to adapt to ischemic stress is rather complex, however, nitric oxide (NO) seems to be an important player [see for a review: Ferdinandy P, Schulz R. Nitric oxide, superoxide, and peroxynitrite in myocardial ischemia-reperfusion injury and preconditioning. Br. J. Pharmacol. 138: 532-543 (2003)].

Although ischemic heart disease in humans is a complex disorder caused by or associated with other systemic diseases and conditions, most experimental studies on cardioprotection have been undertaken in juvenile animal models, in which ischemia/reperfusion is imposed in the absence of other disease processes and risk factors for cardiovascular diseases. Ischemic heart disease develops as a consequence of a number of etiologic risk factors and always co-exists with other disease states. These include systemic arterial hypertension and related left ventricular hypertrophy, hyperlipidemia and atherosclerosis, diabetes and insulin resistance, heart failure and aging. These systemic diseases and aging exert multiple biochemical effects on the heart that can potentially affect the development of ischemia/reperfusion injury per se and interfere with responses to anti-ischemic, cardioprotective interventions.

Accordingly, we have shown that hyperlipidemia leads to the loss of the preconditioning effect in humans as well [Ungi I., Ungi T., Ruzsa Z., Nagy E., Zimmermann Z., Csont T., Ferdinandy P. Hypercholesterolemia attenuates the anti-ischemic effect of preconditioning during coronary angioplasty. Chest 128:1623-1628 (2005)]. Therefore, the development of rational therapeutic approaches to protect the ischemic heart requires preclinical studies that examine cardioprotection specifically in relation to complicating disease states and risk factors such as hyperlipidemia. The mechanism by which hyperlipidemia interferes with the cardioprotective effect of stress adaptation mechanisms includes disruption of NO-dependent pathways due to increased formation of free radicals including peroxynitrite [see for reviews: Ferdinandy P., Szilvassy Z., Baxter GF. Adaptation to myocardial stress in disease states: is preconditioning a healthy heart phenomenon? Trends Pharmacol. Sci. 19:223-9 (1998); Ferdinandy P. Myocardial ischaemia/reperfusion injury and preconditioning: effects of hypercholesterolaemia/hyperlipidaemia. Br. J. Pharmacol. 138:283-5 (2003); Ferdinandy P, Schulz R, Baxter GF. Interaction of cardiovascular risk factors with myocardial ischemia/reperfusion injury, preconditioning and postconditioning. Pharmacol Rev 59:418-458 (2007); and as above].

We have previously shown that hyperlipidemia leads to enhanced peroxynitrite formation and mild contractile dysfunction characterized by elevation of left ventricular end-diastolic pressure [Onody A., Csonka C., Giricz Z., Ferdinandy P. Hyperlipidemia induced by a cholesterol-rich diet leads to enhanced peroxynitrite formation in rat hearts. Cardiovasc. Res. 58:663-70 (2003)]. Ahmed et al. have shown that myocardial biglycan mRNA levels in non-ischemic tissue of both left and right ventricles of heart failure rats were substantially elevated in a rat myocardial infarction model as compared to sham-operated rats. They suggested that byglican expression is related to ischemic heart failure after myocardial infarction and promoted that pharmacological inhibition of expression of biglycan gene have benefitial effect [Ahmed MS., Oie E., Vinge LE., Yndestad A., Andersen G. GO., Andersson Y., Attramadal T., Attramadal H.: Induction of myocardial biglycan in heart failure in rats - an extracellular matrix component targeted by AT(1) receptor antagonism. Cardiovasc. Res. 60:557-568 (2003)].

Another study suggested that increased expression of biglycan after myocardial infarction is related to cardiac fibrosis during the healing process, but no relation of biglycan and cardioprotection was suggested. Moreover, in this study increased biglycan expression could be related to post-infarction cardiac contractile dysfunction [Yamamoto, K., Kusachi, S., Ninomiya, Y., Murakami, M., Doi, M., Takeda, K., Shinji, T., Higashi, T. and others: Increase in the Expression of Biglycan mRNA Expression Co-localized Closely with that of Type I Collagen mRNA in the Infarct Zone After Experimentally-Induced Myocardial Infarction in Rats. J. Mol. Cell. Cardiol. 30, 1749-1756 (1998)].

Here we would underline that on the basis of these relevant prior art, a skilled person should draw the conclusion that an increase in the level of biglycan should facilitate cardiac dysfunction related to acute myocardial infarction, i.e. it should exert a negative effect in this disorder.

The aim of the inventors of the present invention was to further study the effect of biglycan on atherosclerotic diseases and on cardiac dysfunctions, especially on atherosclerotic plaque formation and ischemia reperfusion injury as measured by infarct size in the presence or absence of hyperlipidemia.

### SUMMARY OF THE INVENTION

It has now been surprisingly found that biglycan (see the "Definition of terms" part) shows potent anti-atherosclerotic and anti-ischemic (e.g. cardioprotective) effect. Moreover, according to our experiments, these effects are independent from the presence or absence of hyperlipidemia.

Based on our experiments described below we found that biglycan has an important role in cardiac remodeling and mediates cardioprotection. Moreover, on the basis of the results it can be supposed that addition of biglycan will prevent and treat cells from any injury induced by hypoxia/ischemia and reperfusion/reoxygenation, the treatments of these injuries covered by the term anti-ischemic treatment.

Our aim is to enhance the biglycan activity in the target tissue or organ, preferably in the heart, especially by increasing the local level of biglycan and/or by increasing the specific activity of biglycan by enhancers (see the "Definition of term" part).This can be achieved by one of the following methods:
a) increasing the biglycan expression in the target tissue or organ;
b) introduction of exogenous biglycan gene or protein into the target tissue or organ by
   i) gene therapy: delivery of recombinant DNA either as naked DNA or entrapped into liposomes or packed into adenovirus, retrovirus or baculovirus into the target tissue or organ;
   ii) somatic cell therapy: introduction of myoblast cells overexpressing biglycan protein;
   iii) stem cell therapy: introduction of hematopoietic stem cells or embryonic stem (ES) cells overexpressing biglycan protein;
   iv) delivery of recombinant biglycan protein produced in mammalian cells in vitro or purified from mammalian tissues (e.g. from articular cartilage.) into the target tissue or organ.

Accordingly, the present invention relates to the following subject matters:
1. Use of biglycan or an enhancer of biglycan activity as an active ingredient in the preparation of pharmaceutical compositions for preventing the formation of atherosclerotic plaque.
2. The use according to item 1, wherein the active ingredient is selected from the following group: biglycans, preferably decorin, inducers or activators of biglycan, delivery systems containing a nucleic acid encoding biglycan and nucleic acids encoding biglycan (naked nucleic acid).
3. The use according to item 1, wherein the active ingredient is an activator or inducer of the overexpression of endogenous (native) biglycan.
4. Biglycan or an enhancer of biglycan activity for use as an active ingredient in the prevention of the formation of atherosclerotic plaque..
5. The product for use according to item 4 wherein the active ingredient is selected from the following group: biglycans, preferably decorin, inducers or activators of biglycan, delivery systems containing a nucleic acid encoding biglycan and nucleic acids encoding biglycan (naked nucleic acid).
6. The product for use according to item 5 wherein the active ingredient is an activator or inducer of the overexpression of endogenous (native) biglycan.
7. The product for use according to item 6, wherein the activator or inducer of the overexpression of endogenous (native) biglycan is selected from the group of small molecules, preferably by oligo- or polipeptides or Na-salicylate or oxysterol or lysolipids.
8. The product for use according to item 5, wherein the active ingredient is a gene delivery system containing a nucleic acid encoding biglycan, where said gene delivery system ensures recombinant overexpression of biglycan.
9. The product for use according to any of item 4 to 8, wherein the active ingredient is incorporated into autografts *ex vivo* before implantation in the case of a bypass surgery, preferably coronary bypass surgery.

The term "known auxiliaries" embraces carriers, additives, solutions and other known ingredients which are commonly applied in the preparation of supplements to cell or tissue cultures or to solutions used for storage of organs before transplantation.

### DETAILED DESCRIPTION OF THE INVENTION

The above applicability is based on our experimental results which prove that an increased biglycan activity, preferably coming from the increased level of biglycan has a potent preventive and/or theraputic effect in atherosclerotic diseases which are in connection with the formation of any atherosclerotic plaque, such as coronary sclerosis, carotid artery sclerosis, peripheral artery sclerosis.

As a further advantageous property of the increased biglycan activity, it has preventive and/or therapeutic effect in ischemic diseases, e.g. in cardiac diseases such as acute ischemic heart diseases e.g. acute myocardial ischemia and/or unstable angina and/or myocardial infarction, and/or procedures of cardiac surgery which involve myocardial ischemia. Moreover, the cardioprotective effect involves reduction of infarct size, improvement of post-infarction myocardial function and/or hyperlipidemia-induced cardiac dysfunction. The above effects develop either in the presence and or in absence of hyperlipidemia. These further positive properties ("positive side effects") underline the usability of the active agent for preventing the formation of atherosclerotic plaque. The invention has a remarkable importance since biglycan is an ubiquitous molecule in most tissues, so it should exert its positive effect in the whole body of the treated animal or human being.

### DEFINITION OF TERMS

The most important terms applied in the definition and discussion of the present invention (i.e. in the description and the set of claims) are defined below. The other terms should be applied according to their general meaning known for a skillled person.

### Biglycan

Biglycan is a member of the small leucine rich proteoglycan (SLRP) family [sometimes named as leucine-rich repeat (LRR) protein family] and is composed of a 38kDa core protein that is substituted with two glycosaminoglycan chains on N-terminal Ser-Gly sites. The core protein contains ten leucine rich repeats flanked by disulphide bond stabilized loops on both sides. It contains additional sites for glycosylation (N-linked glycosylation sites) within the leucine-rich repeats. The quality of the glycosaminoglycans varies both with regard to the length and composition. The backbone of the glycosaminoglycan chain is composed of repeating disaccharide units of N-acetylgalactosamine and glucuronic acid, the latter often being converted into iduronic acid through epimerization at carbon 5. As the chains are elongated they are modified by sulphation resulting in chondroitin sulphate and dermatan sulphate respectively. The degree of epimerization and sulphation varies between tissues. An isoform of biglycan with a single glycosaminoglycan substitution also exists (http://www.cmb.lu.se/ctb/html/Biglycan.htm).

As it comes from the above definition, the term "biglycan" embraces a family of similar compounds in case of human beings [human biglycan is described e.g. in Fischer et al. as "bone small proteoglycan" in J. Biol. Chem. 264: 4571 (1989); GenBank Accession No. J04599]. Of course, similar compounds can be found in other animals.

Moreover, the term "biglycan" intends to embrace analogues, derivatives and parts of the above compounds, in line with the definition of biglycan applied in US patent application No. 20050059580. In the following part the most important parts of the general discussion of biglycans of this document are cited (see paragraph 000054 of the specification), but here we would emphasize that the whole document is incorporated into this specification as a reference. For instance a preferred example of such biglycan analogues is the proteoglycan II (decorin).

The term "biglycan" also refers to proteoglycans having at least one biological activity of human or other animal originated biglycan. Preferred biglycans include Torpedo DAG-125 (comprising SEQ ID NO: 1-3 in US patent application No. 20050059580), human biglycan as we as homologs thereof. Preferred homologs are proteoglycans or proteins or peptides having at least about 70% identity, at least about 75% identity, at least about 80% identity, at least about 85% identity, at least about 90% identity, at least about 95% identity, and even more preferably, at least about 98 or 99% identity. Even more preferred homologs are those which have a certain percentage of homology (or identity) with human biglycan or Torpedo DAG-125 and have at least one biological activity of these proteoglycans. The term biglycan is not limited to the full length biglycan, but includes also portions having at least one activity of biglycan.

The term "biglycan" as applied here embraces all the above variants.

### Enhancers of biglycan activity

The desired activity increase can be achived by the use of biglycan or by an enhancer of the biglycan activity which can be selected from the following group:
- an inducer or activator of expression of biglycan,
- a delivery system containing nucleic acid (gene) encoding biglycan and
- a nucleic acid encoding biglycan (naked gene).

### Inducers or activators of expression of biglycan

These terms relate to such substances which enhance the biglycan activity in the target tissue or organ by
a) increasing the expressison of biglycan by inducing expression of biglycan, preferably of the endogenous (native) biglycan; these substances are named as "inducers" or "inducers of biglycan" in the specification; or
b) increasing the specific activity of the biglycan, preferably the endogenous (native) biglycan; these substances are named as "activators" or "activators of biglycan"in the specification.

The term "endogenous (native) expression of biglycan" relates to such expression of biglycan which is a natural function of the human or animal body to be treated or prevented from the disease.

### Inducers of biglycan

The inducers can be e.g.
i) inorganic compounds such as sodium salicylate, small organic molecules, such as oligo- and polypeptides, gene or proteins having a molecular weight less than 5 kD etc.;
ii) cytokines such as transforming growth factor beta (TGF-β) and
iii) co-factors, i.e. endogenous small molecules or peptides necessary for normal or enhanced biglycan activity.

In a preferred embodiment the Inducers of the production of biglycan can be different growth factors and small molecules are known in the art, e.g. *transforming growth factor-beta (TGF-beta)* [Ungefroren H, Groth S, Ruhnke M, Kalthoff H, Fandrich F. Transforming growth factor-beta (TGF-beta) type I receptor/ALK5-dependent activation of the GADD45beta gene mediates the induction of biglycan expression by TGF-beta J. Biol Chem. 280:2644-52 (2005); Kaji, T., Yamada, A., Miyajima, S., Yamamoto, C., Fujiwara, Y., Wight, T.N., and Kinsella, M.G.: Cell Density-dependent Regulation of Proteoglycan Synthesis by Transforming Growth Factor-beta 1 in Cultured Bovine Aortic Endothelial Cells. J. Biol. Chem. 275, 1463-1470 (2000)], *oxysterols* (e.g. 7-ketocholesterol and 7-beta-hydroxycholesterol) and *lysolipids* (e.g. lysophosphatidylcholine and lysophosphatidic acid) [Chang MY, Tsoi C, Wight TN, Chait A. Lysophosphatidylcholine regulates synthesis of biglycan and the proteoglycan form of macrophage colony stimulating factor. Arterioscler Thromb Vasc Biol. 23:809-15 (2003)] and *salicylate,* preferably Na-salicylate [Silva AM, Reis LF. Sodium salicylate induces the expression of the immunophilin FKBP51 and biglycan genes and inhibits p34cdc2 mRNA both in vitro and in vivo. Biol Chem. 275(46):36388-93 (Nov 17, 2000)] have been shown to induce biglycan.

In general terms the inducers can be inorganic compounds or organic compounds like proteins, nucleic acids (genes), peptides (e.g. oligo- and polypeptides), peptidomimetics, carbohydrates or lipids. Chemical libraries containing such compounds can be screened for identifying inducers of the biglycan expression by known screening technics (e.g high throughput analysis) or by the *in vitro* screening method according to the present invention.

Preferably the inducers are "small" organic molecules, i.e. molecules having molecular weight less than 5 kD, preferably less than 4 kD, more preferably less than 3 kD.

### Activators of biglycan

Activators of biglycan can be e.g. substances which enhance the formation of the active biglycan from inactive biglycan or enhance the activity of the biglycan by any other method. For example, if the specific embodiment of the biglycan is a biglycan core protein, then it can be activated by a post-translational glycolization.

In genereal terms the activators can be inorganic compounds or organic compounds like proteins, nucleic acids (genes), peptides (e.g. oligo- or polypeptides), peptidomimetics, carbohydrates or lipids.. Chemical libraries containing such compounds can be screened for identifyingenhancers of the biglycan activity by known screening technics (e.g high throughput analysis) or by the screening method according to the present invention.

Preferably the activators are "small" organic molecules, i.e. molecules having molecular weight less than 5 kD, preferably less than 4 kD, more preferably less than 3 kD.

### Target organ or tissue

The term "target organ or tissue" relates to a part of a human or animal body where the disease should be prevented or treated, it can be e.g. heart, kidney, liver, skeletal muscle, skin, brain tissue. In case of cardioprotective effect it is in the heart, preferably it is such part of the heart where an ischemic attack may happen or occurred, or the neighboring part thereof. In case of anti-atherosclerotic effect it is that part of a human or animal body where the formation of atherosclerotic plaque may happen or occurred, or the neighboring part thereof.

### Recombinant DNA

Recombinant DNA means such a DNA which was produced by recombinant technique. It means that this phrase embraces the native gene, too, if it was produced by recombinant technique.

### Recombinant protein/biglycan

Recombinant protein/biglycan means such a protein/biglycan which was expressed from a gene produced by recombinant technique. It means that this phrase embraces the native protein/biglycan, too, if it was expressed from the native gene produced by recombinant technique.

### Gene therapy

The term embraces the first group of methods applicable for introduction of exogenous biglycan gene or protein into the target tissue or organ.

This phrase embraces any delivery of a recombinant gene encoding biglycan to the target tissues, either by a delivery system containing the gene encoding biglycan (i.e. in recombinant vector or liposoma or packed into a virus) or even in a naked gene form.

A preferred embodiment of the gene therapy is the expression of recombinant biglycan in the target tissue or organ. This embodiment relates to such expression of biglycan which is a not a natural function of the human or animal body to be treated or prevented from the disease. The applicable methods are detailed in US patent application No. 20050059580. In the following part the most important parts of the general discussion of the methods are cited (see paragraphs 0079 to 0081 of the specification), but here we would emphasize that the whole document is incorporated into this specification as a reference.

As used herein, the term "transfection" means the introduction of a nucleic acid, e.g. an expression vector, into a recipient cell by nucleic acid-mediated gene transfer. The term "transduction" is generally used herein when the transfection with a nucleic acid is by viral delivery of the nucleic acid. "Transformation", as used herein, refers to a process in which a cell's genotype is changed as a result of the cellular uptake of exogenous DNA or RNA, and, for example, the transformed cell expresses a recombinant form of a polypeptide or, in the case of anti-sense expression from the transferred gene, the expression of a naturally-occurring form of the recombinant protein is disrupted.

As used herein, the term "transgene" refers to a nucleic acid sequence which has been introduced into a cell. Daughter cells deriving from a cell in which a transgene has been introduced are also said to contain the transgene (unless it has been deleted). A transgene can encode, e.g. a polypeptide, partly or entirely heterologous, i.e. foreign to the transgenic animal or cell into which it is introduced, or is homologous to an endogenous gene of the transgenic animal or cell into which it is introduced, but which is designed to be inserted, or is inserted into the animal's genome in such a way as to alter the genome of the cell into which it is inserted (e.g. it is inserted at a location which differs from that of the natural gene). Alternatively, a transgene can also be present in an episome. A transgene can include one or more transcriptional regulatory sequences and any other nucleic acid (e.g. intron), that may be necessary for optimal expression of a selected coding sequence.

As used herein, the term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The gene encoding the proteoglycan of the invention can be under the control of a constitutive, or inducible promoter. These molecular biological terms are well known in the art.

Some other gene therapy techniques disclosed in US patent application No. 20050059580 can be applied, too, see. e.g. retrovirus-based, adenovirus-based vectors (see especially paragraphs 0132 to 0139 of the specification).

Moreover, non-viral methods also can be employed to cause expression of protein in the tissue of a human or animal. The applicable methods are detailed in US patent application No. 20050059580. In the following part the most important parts of the general discussion of the methods are cited (see paragraphs 0140 to 0145 of the specification), but here we would emphasize that the whole document is incorporated into this specification as a reference.

Most nonviral methods of gene transfer rely on normal mechanisms used by mammalian cells for the uptake and intracellular transport of macromolecules. In preferred embodiments, non-viral gene delivery systems of the present invention rely on endocytic pathways for the uptake of the gene by the targeted cell. Exemplary gene delivery systems of this type include liposomal derived systems, poly-lysine conjugates, and artificial viral envelopes.

In a representative embodiment, a gene encoding a protein of interest can be entrapped in liposomes bearing positive charges on their surface (e.g., lipofectins) and (optionally) which are tagged with antibodies against cell surface antigens of the target tissue (Mizuno et al., (1992) No Shinkei Geka 20:547-551; PCT publication WO91/06309; Japanese patent application 1047381; and European patent publication EP-A-43075). For example, lipofection of muscle, neural or cardiac cells can be carried out using liposomes tagged with monoclonal antibodies against specific tissue-associated antigens (Mizuno et al., (1992) Neurol. Med. Chir. 32:873-876).

In yet another illustrative embodiment, the gene delivery system comprises an antibody or cell surface ligand which is cross-linked with a gene binding agent such as poly-lysine (see, for example, PCT publications WO93/04701, WO92/22635, WO92/20316, WO92/19749, and WO92/06180). For example, any of the subject gene constructs can be used to transfect specific cells in vivo using a soluble polynucleotide carrier comprising an antibody conjugated to a polycation, e.g. poly-lysine (see U.S. Pat. No. 5,166,320). It will also be appreciated that effective delivery of the subject nucleic acid constructs via-mediated endocytosis can be improved using agents which enhance escape of the gene from the endosomal structures. For instance, whole adenovirus or fusogenic peptides of the influenza HA gene product can be used as part of the delivery system to induce efficient disruption of DNA-containing endosomes (Mulligan et al., (1993) Science 260-926; Wagner et al., (1992) PNAS USA 89:7934; and Christiano et al., (1993) PNAS USA 90:2122).

Nucleic acids encoding biglycan proteins can also be administered to a subject as "naked" DNA, as described, e.g., in U.S. Pat. No. 5,679,647 and related patents by Carson et al., in WO 90/11092 and Felgner et al. (1990) Science 247: 1465.

In clinical settings, the gene delivery systems can be introduced into a patient by any of a number of methods, each of which is familiar in the art. For instance, a pharmaceutical preparation of the gene delivery system can be introduced systemically, e.g. by intravenous injection, and specific transduction of the construct in the target cells occurs predominantly from specificity of transfection provided by the gene delivery vehicle, cell-type or tissue-type expression due to the transcriptional regulatory sequences controlling expression of the gene, or a combination thereof. In other embodiments, initial delivery of the recombinant gene is more limited with introduction into the animal being quite localized. For example, the gene delivery vehicle can be introduced by catheter (see U.S. Pat. No. 5,328,470) or by stereotactic injection (e.g. Chen et al., (1994) PNAS USA 91: 3054-3057).

In the clinical settings, recombinant biglycan can be introduced by coronary catheter into the coronary arteries in acute myocardial infarction during percutaneous coronary intervention.

When the recombinant gene is introduced by catheter it can be directed into the coronary arteries during percutaneous coronary intervention.

### Somatic cell therapy

The term embraces the second group of methods applicable for introduction of exogenous biglycan gene or protein into the target tissue or organ.

In a preferred embodiment the production of active recombinant biglycan expressing cardiac cells and their introduction into cardiac tissues can be carried out by the following methods.
i) Cardiac cells from 16 day-old embryos can be isolated and maintained in cell culture. Cultured cardiac cells can be transformed with recombinant DNA encoding for biglycan protein. The transformation efficiency can be very high (up to 80%) therefore no subsequent selection is needed, and transformed cells can be readily reintroduced into cardiac tissues.
ii) Using embryonic stem cells: There are well established methods to isolate embryonic stem cells (ES) from different mammalian blastocysts. These blastocysts derived ES-cells can be maintained in culture and can be transformed by electroporation with different recombinant DNA construct. The transformed cells - under appropriate conditions - can be differentiated to myoblast cells in vitro [Sachinidis A, Fleischmann BK, Kolossov E, Wartenberg M, Sauer H, Hescheler J. Cardiac specific differentiation of mouse embryonic stem cells. Cardiovasc Res., 58:278-91 (2003); Guo XM, Wang CY, Tian XC, Yang X. Engineering cardiac tissue from embryonic stem cells. Methods Enzymol.; 420:316-38 (2006)] Then, these transformed myoblast cells overexpressing the biglycan protein can be reintroduced into the myocardium or the coronary arteries.

### Stem cell therapy

The term embraces the third group of methods applicable for introduction of exogenous biglycan gene or protein into the target tissue or organ using one of the following methods:
*Using embryonic stem cells:* There are well established methods to isolate embryonic stem cells (ES) from different mammalian blastocysts. These blastocysts derived ES-cells can be maintained in culture and can be transformed by electroporation with different recombinant DNA construct. The transformed cells can be selected in the appropriate antibiotic solution and cloned. Cloned ES cells overexpressing the biglycan protein can be introduced into the myocardium or coronary arteries. The differentiation of biglycan producing ES cells will occur *in situ* in the heart by different growth factors.
*Using hematopoietic stem cells:* Hematopoietic stem cells can be isolated from bone-marrow, maintained in vitro using standard methods and transformed with recombinant biglycan construct. Transformed hematopoietic stem cells overexpressing biglycan protein can be injected into the vein, where they start to migrate towards the heart attracted by different growth factors released by the injured cardiac cells. If the biglycan is tagged with a fluorogenic molecule or fused to fluorescence protein, such as EGFP or DsRed, accumulation and differentiation of biglycan producing hematopoietic stem cells can be monitored based on their fluorescence.

### Delivery of recombinant or native purified biglycan protein

The term embraces the fourth group of methods applicable for introduction of exogenous biglycan gene or protein into the target tissue or organ.

Accordingly, in a preferred embodiment biglycan protein produced by recombinant DNA technique in mammalian cells or purified from mammalian cartilages can be injected into the vascular system of the target organ. Other administration routes are also applicable, e.g. by subcutaneous injections or implants.

In mammalian cells the biglycan undergoes a posttranslational modification and two chrondroitin/dermatan sulfate glycosaminoglycan chains are added to the core protein. Recombinant biglycan protein can be produced in myoblast cell culture (i.e. H9C2) after transfection with CMV-biglycan recombinant DNA construct and subsequent selection in G418. 6xHis-tagged biglycan protein can be purified rapidly from myoblast cells using Ni-NTA resin [Crowe J, Masone BS, Ribbe J.. One-step purification of recombinant proteins with the 6xHis tag and Ni-NTA resin. Mol Biotechnol. 1995. Dec;4(3):247-58].

Native biglycan protein can be purified from chondrocytes of different mammalian species using the following methods described elsewhere [Pogany G, Hernandez DJ, Vogel KG. The in vitro interaction of proteoglycans with type I collagen is modulated by phosphate. Arch Biochem Biophys. 1994 Aug 15;313(1):102-11; Schonherr E, Witsch-Prehm P, Harrach B, Robenek H, Rauterberg J, Kresse H. Interaction of biglycan with type I collagen. J Biol Chem. 1995 Feb 10;270(6):2776-83.; Roughley PJ, Melching LI, Recklies AD. Changes in the expression of decorin and biglycan in human articular cartilage with age and regulation by TGF-beta. Matrix Biol. 1994 Jan;14(1):51-9.]

In an other preferred embodiment a bypass graft or any vein autograft can be treated *ex vivo* with the above mentioned methods before implantation.

### Screening Methods

The invention also provides a screening method by which enhancers of biglycan activity can be identified.

In a preferred embodiment such levels of genes or protein are applied for identification in the said basic cells which are specific to the anti-atherosclerotic and/or cardioprotective effect of biglycan. In this case the detected expression levels are compared to the specific levels obtained in the basic cell without the use of the candidate moledule or in other cells overexpressing the biglycan. These specific levels are appropriate to select those candidates which exert the desired anti-atherosclerotic and/or cardioprotective effect, respectively.

The term "candidate" embraces any substances which can be applied for enhancing the activity of biglycan, see especially the substances enumerated under the definition of biglycan, Inducers or activators of expression of biglycan and tools of gene therapy.
i) In a preferred embodiment inducers of biglycan expression : the screening method is based on an *in vitro* reporter-assay, wherein a mammalian cell-line (preferable myoblast cell-culture, such as H9C2) is stable transformed by a reporter-gene driven by biglycan promoter. The reporter gene can be any easily identifiable genes such as EGFP, DsRed, luciferase or chloramphenicol acetyl transferase (CAT), etc. Upon induction of biglycan expression by small molecules the cells will emit green (EGFP) or red (DsRed) fluorescence, luminescence (luciferase) or enhanced enzyme activity can be quantatively measured by radioactivity (CAT). Cellular reporter-assay can be performed by inserting the reporter gene into large genomic locus (episomes, artificial chromosomes) under the control of biglycan promoter (called genomic reporter assays, GRA) as described earlier for fetal hemoglobin [Vadolas J, Wardan H, Orford M, Williamson R, Ioannou PA. Cellular genomic reporter assays for screening and evaluation of inducers of fetal hemoglobin. Hum Mol Genet., 13(2):223-33 (Jan 15, 2004)]. Insertion of a reporter gene into the endogenous biglycan locus, under the control of biglycan promoter by homologous recombination is a further alternative.
ii) Enhancers of the specific activity of biglycan: these screening method are based on the identification of proteins which are able to interact or/and bind to biglycan protein and enhance its activity either by glycolization or causing a conformational change in the molecule. The protein-protein interaction can be studied by the classical yeast two-hybrid system. Another high-throughput method is the use of protein chips. The biglycan protein can be anchored to glass surface and cardiac cell-lysates derived from drug-treated cardiac/myoblast cells can be applied to the chip. After subsequent washing steps proteins bound to the biglycan can be identified. Then, enhancer activity of purified proteins can be tested *in vitro* in cardiac cell culture.

### Supplement to culture or storage solution

The invention also provides a supplement to a cell or tissue culture or to a solution which can be used for storage of organs before transplantation, which contains an enhancer of biglycan activity.

The amount of compound to be added to the supplement can be determined in small scale experiments, e.g. by incubating the cells or organs with increasing amounts of a specific enhancer of the invention. Preferred cells include eukaryotic cells, e.g. muscle cells, neuronal cells-and cardiac cells.

### Pharmaceutical compositions

According to the present invention the enhancers of biglycan activity can be applied together with pharmaceutically acceptable auxiliaries and optionally other therapeutic agents. The term "acceptable" means being compatible with the other ingredients of the composition and not deleterious to the recipient thereof.

Said pharmaceutical compositions may be prepared by conventional methods of pharmacy, e.g. as described in any standard reference, e.g. Gennaro et al., Remington's Pharmaceutical Sciences (18th ed., Mack Publishing Company, 1990, see especially Part 8: Pharmaceutical Preparations and Their Manufacture). Examples of pharmaceutical compositions are tablets, capsules, pills, sachets, liquid forms for oral administration, such as solutions, suspensions and emulsions; controlled release forms for oral administration or enteric administration in general; forms for parenteral administration, such as injectable forms.

### BRIEF DESCRIPTION OF THE DRAWINGS

Figure 1 shows the mRNA expression of biglycan in the aorta and heart of transgenic and control mice analyzed by QRT-PCR.
   Meanings of the abbreviations:
   C: control (wild-type animals);
   Bg^{+/+}: biglycan overexpression.
Figure 2 shows Western analysis of iNOS, eNOS and nNOS (part A), pyk2 and synaptotagmin (part B) in control and transgenic heart samples; expressions were normalized to the endogeneous mouse β-actin level.
   Meanings of the abbreviations:
   C: control (wild-type animals);
   β-act: β-actin;
   Tg: transgenic animal.
Figure 3 shows serum cholesterol levels in test animals.
Figure 4 shows aortic flow values in different test animals.
Figure 5 shows myocardial superoxide levels in different test animals.
Figure 6 shows aortic plaque area values in different test animals.
   Meanings of the abbreviations in Figures 3 to 6:
   Control: wild-type animals;
   Chol.: animals treated with high cholesterol diet;
   ApoB: ApoB transgenic animals.
Figure 7 shows the cytoprotective effect (based on anti-ischemic effect) of different concentrations of exogenous biglycan in cardiac myocytes subjected to hypoxia and reoxygenation. Figure 8 shows the cytoprotective effect of different concentrations of exogenous biglycan core protein in myocardial cells subjected to hypoxia and reoxygenation
Figure 9 shows the cytoprotective effect of different concentrations of exogenous biglycan analogue decorin in myocardial cells subjected to hypoxia and reoxygenation.
   Meanings of the abbreviations in Figures 7 to 9:
   BGN: biglycan;
   *: p<0.05 versus hypoxic controls (ANOVA followed by LSD post-hoc test; in case of fig 8, the Hypoxic BGN-core 100 nM group was not included in the statistics, due to high variation in this group)

The present invention is further illustrated by the following examples.

### Example 1

### Generation of biglycan transgenic preparations and induction of vasculo- and cardioprotective cellular transduction pathways in the heart by biglycan

In this study we generated transgenic mice overexpressing the human biglycan gene. Cardiac protein profile of transgenic offsprings was investigated using proteomics.

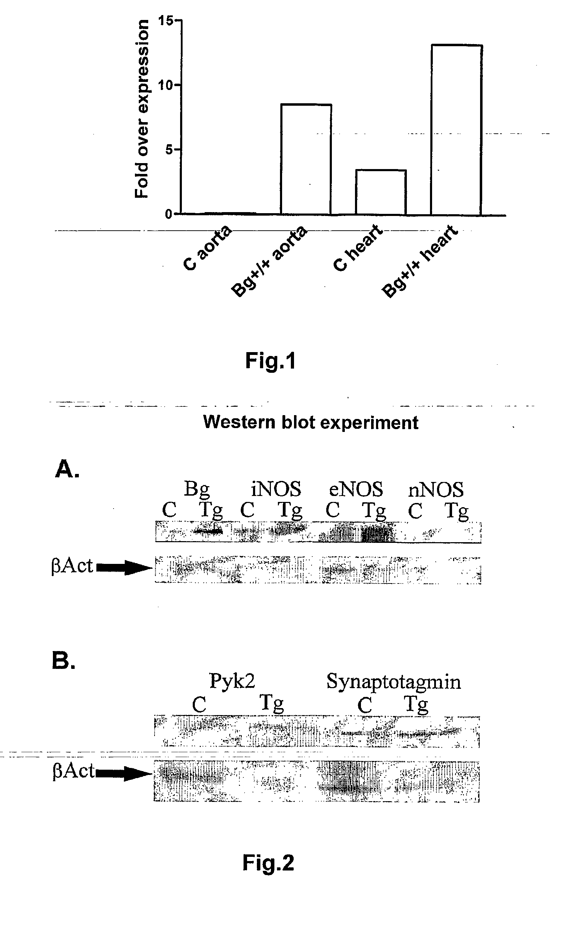

The transgenic construct contained the human biglycan cDNA fused to a CMV promoter. A V5 epitope and a 6x His Tag sequence were also fused to the 3' end of cDNA. Newborns were genotyped using PCR and transgene expression from different tissues (liver, brain, heart and muscle) of PCR positive transgenic mice was tested using Taqman probe based quantitative real-time PCR (QRT-PCR) (data not shown). Transgenic line 1052, expressing the highest level of human biglycan mRNA was selected for further study. Expression of human biglycan at mRNA level in the aorta and heart of transgenic and age-matched control mice was assessed by QRT-PCR (Fig.1).

Using QRT-PCR and antibody array we detected elevated gene expression level of TGFβ1 and TGFβ2 (data not shown), and elevated protein level of Pyk2, RAF-1 and mcl-1 (Table I.). Biglycan binds to TGF β with a high affinity. TGF β promotes the synthesis of biglycan in both dense and sparse endothelial cells [Kaji, T., Yamada, A., Miyajima, S., Yamamoto, C., Fujiwara, Y., Wight, T.N., and Kinsella, M.G.: Cell Density-dependent Regulation of Proteoglycan Synthesis by Transforming Growth Factor-beta 1 in Cultured Bovine Aortic Endothelial Cells. J. Biol. Chem. 275, 1463-1470 (2000)]. It was recently shown that induction of myocardial biglycan may shift this equilibrium of interaction and enhance TGF β activity in the myocardial tissue [Ahmed, M.S., Oie, E., Vinge, L.E., Yndestad, A., Andersen, G.O., Andersson, Y., Attramadal, T., and Attramadal, H.: Induction of myocardial biglycan in heart failure in rats--an extracellular matrix component targeted by AT1 receptor antagonism. Cardiovasc. Res. 60, 557-568 (2003)]. Heterodimerisation with TGF β may affect both the availability (in the local concentration) and the activity of TGF β. TGF β is an important mediator of fibroblast proliferation and of synthesis of extracellular matrix components, particularly collagen and fibronectin [de Andrade, C.R., Cotrin, P., Graner, E., Almeida, O.P., Sauk, J.J., and Coletta, R.D.: Transforming growth factor -beta1 autocrine stimulation regulates fibroblast proliferation in hereditary gingival fibromatosis. J. Periodontol. 1726-1733 (2001)]. There are several data that point out the importance of TGF β in myocardial remodeling and fibrosis in heart failure [Lijnen, P.J., Petrov, V.V., and Fagard, R.H.: Induction of Cardiac Fibrosis by Transforming Growth Factor-[beta]1. Mol. Gen. Metabol. 71, 418-435 (2000)].

To identify proteins with altered expression in the heart of biglycan transgenic mice Panorama Ab Microarray Cell Signaling Kit (Sigma, CSAA1) was used. This array contained 224 antibodies to cellular proteins involved in apoptosis, cell cycle, cellular stress and signal transduction. Other groups of antibodies were against structural proteins (nuclear, cytoskeletal, and neuron specific). Upregulation of pyk2 protein was detected using protein profiling of cardiac tissues of biglycan transgenic mice. These proteins are key molecules of different signaling pathways playing a crucial role in cell survival and apoptotic cell death. Pyk2, also known as related adhesion focal tyrosine kinase (RAFTK), plays a crucial role in cardiac remodeling and in activation of the apoptotic pathway in cardiomyocytes through Src kinase-p38 downstream signaling [Melendez, J., Turner, C., Avraham, H., Steinberg, S.F., Schaefer, E., and Sussman, M.A.: Cardiomyocyte Apoptosis Triggered by RAFTK/pyk2 via Src Kinase Is Antagonized by Paxillin. J. Biol. Chem. 279, 53516-53523 (2004)]. Although phosphorylation of kinases involved in apoptotic signalling was not investigated during this study, p38 as well as other mitogen-activated protein kinase (MAPK) members of apoptotic pathway were shown to be upregulated in the antibody microarray study. It has been previously reported that biglycan mRNA level is increased in the myocardial zone affected by infarction, suggesting a pivotal role of biglycan during healing of the infarcted area and indicating its involvement in cardiac remodeling [Yamamoto, K., Kusachi, S., Ninomiya, Y., Murakami, M., Doi, M., Takeda, K., Shinji, T., Higashi, T. and others: Increase in the Expression of Biglycan mRNA Expression Co-localized Closely with that of Type I Collagen mRNA in the Infarct Zone After Experimentally-Induced Myocardial Infarction in Rats. J. Mol. Cell. Card. 30, 1749-1756 (1998)]. Here, we also show that another protein involved in Ca++ sensing, synaptotagmin (syt), is also upregulated in the heart of biglycan transgenic mice. Syt is a transmembrane protein containing tandem calcium-binding C2 domains (C2A and C2B) and plays an important role in synaptic transmission. synaptotagmin I is thought to be the fast calcium sensor for synchronous neurotransmitter release [Yoshihara, M., Adolfsen, B., and Littleton, J.T.: Is synaptotagmin the calcium sensor? Curr. Opin. Neurobiol. 13, 315-323 (2003)].

Results obtained by antibody array study are summarized in Table I..

**Table I. List of antibodies of Panorama™ Ab Microarray, which recognize induced protein expressions in biglycan transgenic heart.**

| Spot | Antibody name | Sigma Acc. NO. | Fold over-expression |
|---|---|---|---|
| 5.1Dab | i-NOS | N7782 | 1.67 |
| 5.2Dab | n-NOS | N7155 | 2.20 |
| 5.3Dab | e-NOS | N2643 | 2.14 |
| 6.3Cab | Synaptotagmin | S2177 | 1.83 |
| 7.4Cab | Mcl-1 | M8434 | 1.83 |
| 8.1Dab | Pyk2 | P3902 | 1.99 |

Elevated level of all three types of NOS proteins in the heart of bg^{+/+} transgenic mice were detected. These increases were 2.2 fold for nNOS, 2.14 fold for eNOS, and 1.67 fold for iNOS. Among proteins exerting a role in neurobiological processes, elevated level of synaptotagmin (1.83 fold), was detected. It is known that high level of mcl-1 promotes cell survival while its downregulation initiates apoptosis. From signal transduction proteins present on the array, among other proteins we found marked elevation for Mcl-1 (1.83 fold) and Pyk 2 (1.99 fold).

For validating the results obtained by antibody microarray studies western blot experiments were performed (part A of and part B of Fig. 2). In the first experiment expression levels of iNOS, eNOS and nNOS from control and transgenic mice heart were compared. An approximately 2 fold overexpression was detected for eNOS, 1.9 fold for iNOS, and 1.7 fold for nNOS in transgenic cardiac tissues, compared to control samples and normalized to endogenous β-actin expressions. Elevated protein level of synaptotagmin (2.05 fold) and pyk 2 (2 fold) were found (Fig. 2.B). These western blot results confirmed the previous findings obtained by microarray analysis.

Immunohistochemical analysis on cardiac frozen sections were also performed in order to visualize the localization of pyk2, synaptotagmin and the NOS proteins. Myocardial tissue and endothelial cells showed increased immunoreactivity for eNOS and iNOS in biglycan transgenic heart sections (data not shown)

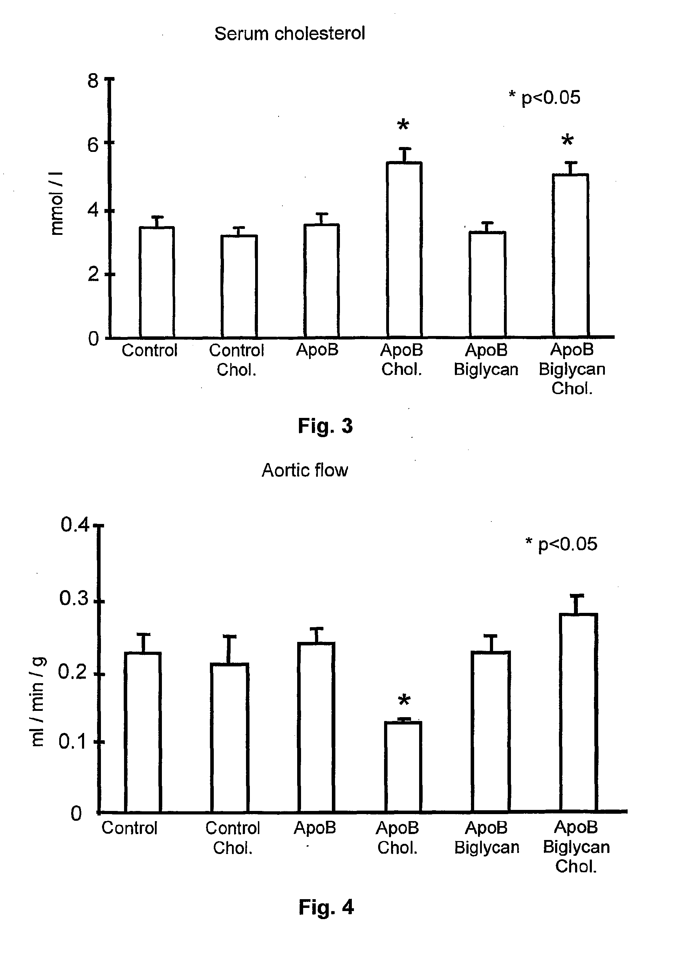

Abundant staining of eNOS in transgenic endothelium was detected. iNOS was localized to the cytoplasm throughout the myocardium. While its distribution did not vary, the intensity was markedly increased in transgenic heart sections. Synaptotagmin and Pyk 2 showed a diffuse staining throughout the cytoplasm (Fig.3.B.), nevertheless both of them showed increased immunoreactivity in the biglycan transgenic heart sections, with an accumulation of pyk2 in the intercellular junctions.

In this study it was shown that using proteomics induced expression of all three types of NOS can be detected in the heart of biglycan transgenic mice. These data were also confirmed by western blot analysis and immunohistochemistry. Nitric oxide being an important signalling molecule plays a crucial role in a variety of biological processes like neurotransmission, cardioprotection and immune defence. NO has cardioprotective effect via its vasodilatator, antioxidant, antiplatelet, and antineutrophil actions and it is essential for normal heart function albeit, overproduction of NO may become toxic for the cells [see for a review: Ferdinandy P, Schulz R. Nitric oxide, superoxide, and peroxynitrite in myocardial ischemia-reperfusion injury and preconditioning. Br. J. Pharmacol. 138: 532-543 (2003)]. Excess of local NO might inhibit biglycan expression as this negative regulation was demonstrated earlier by Schaefer et al [see above]. We found elevated levels of proteins with important role in cardioprotection, induced in our biglycan transgenic mice, underlying the importance of biglycan in cardiac remodeling and its-possible cardioprotective role.

In conclusion, our present study revealed that overexpression of biglycan protein in the heart leads to multiple alterations in the protein profile of cardiac tissues, involving mechanisms of cardiac remodelling, signal transduction, cardioprotection, and Ca⁺⁺ signalling.

### Example 2

### Overexpression of biglycan prevents atherosclerosis and in hyperlipidemia-induced cardiac dysfunction

This example shows that the biglycan transgene reduces the development of experimental atherosclerosis and attenuates hyperlipidemia-induced myocardial dysfunction.

### Methods:

Wildtype (control, C57/B6 x CBA), transgenic mice overexpressing only human apoB-100 protein (apoB), and double transgenic mice overexpressing both human apoB-100 and biglycan (apoB x BG) were fed either a laboratory chow enriched with 2% cholesterol or a standard chow for 17-19 weeks. At the end of the diet period, hearts were isolated for measurement of basal cardiac function (isolated working heart preparation) and biochemical parameters (measurement of superoxide with lucigenin chemiluminescence), and aortae were removed for lipid staining. Blood samples were collected and assayed for serum lipids, and glucose [see detailed description of the methods used in this example: Onody A, Csonka C, Giricz Z, Ferdinandy P. Hyperlipidemia induced by cholesterol-rich diet leads to enhanced peroxynitrite formation in rat hearts. Cardiovasc Res 58: 663-670 (2003). To determine myocardial contractile function, aortic flow was measured.

### Results:

Neither cholesterol-enriched diet, nor apoB-100 transgene alone or in combination with biglycan affected serum total cholesterol and LDL cholesterol when compared to normal diet-fed wildtype mice (Figure 3). However, serum cholesterol and LDL cholesterol were increased significantly due to cholesterol-enriched diet in the apoB transgenic and apoB x BG double transgenic animals (Figure 4). On the other hand, neither of the treatments affected HDL cholesterol, and serum glucose levels significantly. ApoB transgene alone or in combination with biglycan markedly increased the level of triglycerides in the serum compared to normal diet-fed wildetypes, however, serum triglyerides were reduced significantly by cholesterol in the transgenic mice.

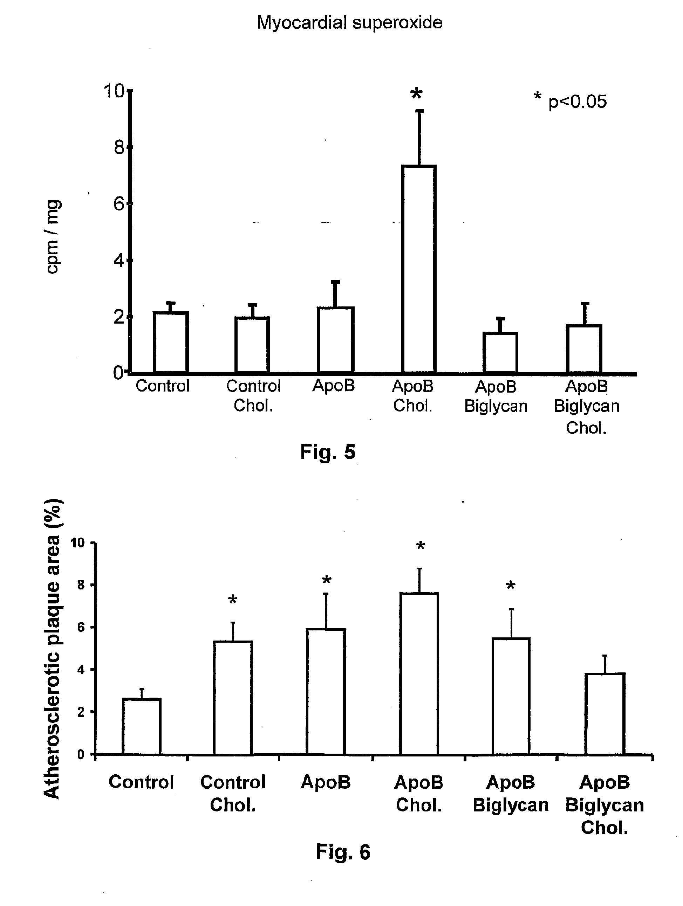

Aortic flow was determined to estimate cardiac performance of the isolated mouse hearts. Neither the apoB transgene nor the apoB x BG double transgene influenced aortic flow when the animals recieved normal diet (Figure 5). However, cholesterol-enriched diet significantly deteriorated aortic flow in the human apoB-100 transgenic mice without having an effect in wildetypes or in apoB x BG transgenes (Figure 4).

Myocardial superoxide content as a major factor for hyperlipidemia-induced cardiac dysfunction [see Onody et al. (2003), shown above] was measured to examine if the biglycan transgene may reduce superoxide production in the myocardium. Cardiac superoxide was markedly increased by cholesterol-enriched diet in the apoB mice, but not in wildtypes and in apoB x BG transgenes. ApoB transgene alone or apoB x BG double transgene did not change cardiac superoxide in mice fed normal diet (Figure 5).

Atherosclerotic plaque area in the aorta was found to be increased in apoB mice compared to wildtype mice. Cholesterol enriched diet further increased plaque formation in the apoB transgenic mice. Although plaque formation in the apoBXbiglycan double transgenic mice was increased, cholesterol enriched diet decreased significantly the atherosclerotic plaque area in the double transgenic animals (Figure 6).

### Conclusions:

This is the first demonstration that the presence of biglycan transgene reduces the development of atherosclerosis and attenuates hyperlipidemia-induced myocardial dysfunction.

### Example 3

### Overexpression of biglycan reduces myocardial ischemia/reperfusion injury including infarct size and postinfarction cardiac dysfunction

This example shows that the presence of the biglycan transgene reduces infarct size and attenuates post-infarction contractile failure in mice hearts subjected to ischemia and reperfusion.

### Methods:

The hears of the wildtype control and transgenic animals overexpressing biglycan gene were isolated and perfused with Krebs-Henselit solution and subjected to 30 min ischemia and 120 min reperfusion. At the end of the perfusion, 5 mL of 1% triphenyltetrazolium-chloride (TTC, Sigma-Aldrich) dissolved in phosphate buffer (pH 7.4) was slowly administeredfor 5 min into the aorta to stain the myocardium. TTC-stained hearts were frozen (-20 °C), cut into approximately 1 mm thick slices, and scanned between glass plates. TTC-stained red and unstained pale areas of images were quantified by planimetry. Infarct size was represented as a percentage of total heart volume [detailed description of the methods used in this example is given: Giricz Z, Lalu MM, Csonka C, Bencsik P, Schulz R, Ferdinandy P. Hyperlipidemia attenuates the infarct-size limiting effect of ischemic preconditioning: role of matrix metalloproteinase-2 inhibition. J Pharmacol Exp Ther, 316:154-161 (2006)]

Results: The presence of biglycan transgene led to an approximately 25-35%, reduction in infarct size [infarct size decreased from 61.2±4.5% in the wildtype group to 44.1±5.8% in the transgenic group (n=6 in both groups)] and approximately 20% improvement of postischemic myocardial function in both cholesterol fed and normal animals (these experiments were carried out in a smaller number of animals).

Conclusions: This is the first demonstration that biglycan exerts cardioprotective effect (based on anti-ischemic effect) which includes reduction of infarct size and improvement of post-ischemic myocardial function.

### Example 4

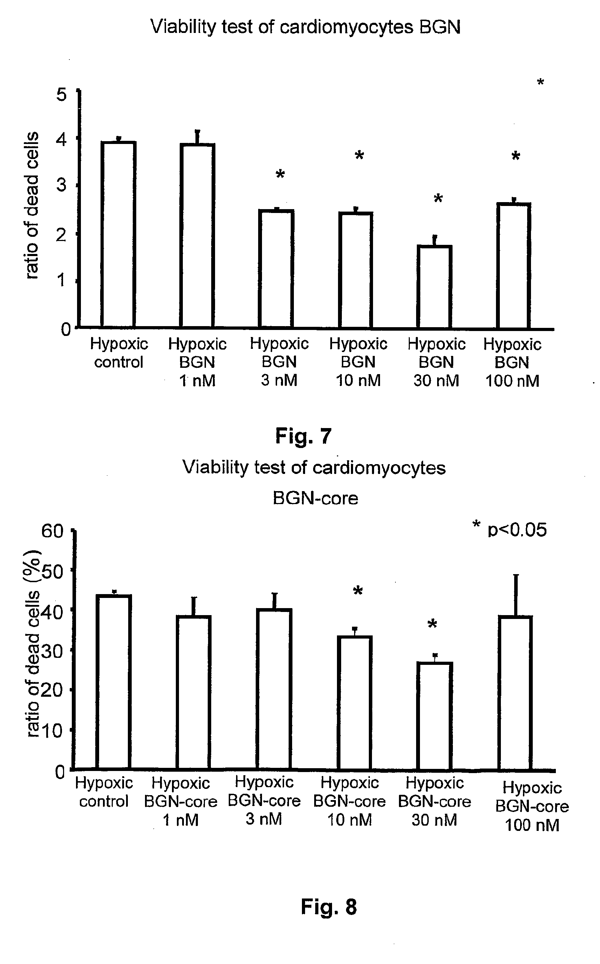

***Biglycan protects myocardial cells from hypoxia*/*****reoxygenation injury** (model for* ***ischemia*/*reperfusion injury)*** Methods: Isolated neonatal rat cardiomyocytes in primary culture were subjected to 2.5 h simulated ischemia and 2 h re-oxygenation and the extent of irreversible cell injury was assessed using trypan blue uptake as described [Li X, Heinzel FR, Boengler K, Schulz R, Heusch G. Role of connexin 43 in ischemic preconditioning does not involve intercellular communication through gap junctions. J Mol Cell Cardiol. 36:161-163, 2004]. Cells were treated with a series of concentration 1 nM-100 nM of biglycan core protein (BGN-core) and full biglycan (BGN) for 20 hours before hypoxia/reoxygenation. The number of the repeated experiments (n) in case of biglycan (Figure 7) were as follows: n=14 in the hypoxic group, n=5 in BGN 1, 3 and 30 nM groups, respectively, n=12 in BGN 10 nM group and n=12 in BGN 100 nM group. The number of the repeated experiments (n) in case of biglycan core protein (Figure 8) were as follows: n=6 in the hypoxic group, n=5 in BGN 1, 3, 10 and 30 nM groups, respectively and n=4 in BGN 100 nM group.

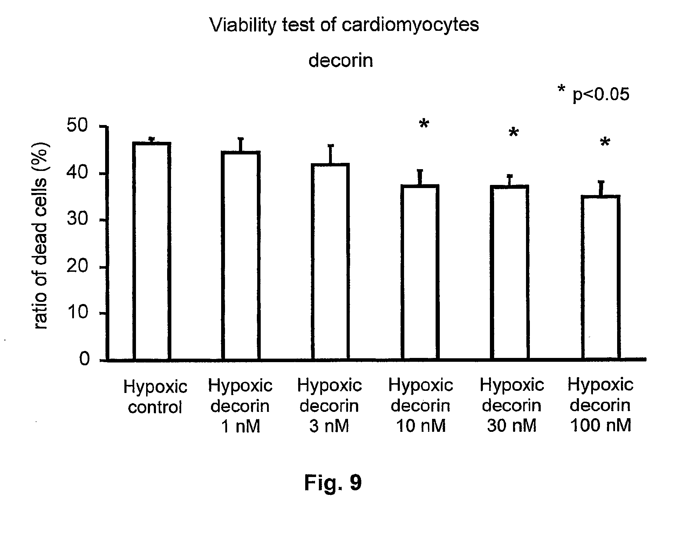

In addition, cells were treated with decorin, another SLRP having a chemical structure similar to that of biglycans but containing only one glucoseaminoglycan side chains [Scott PG, Dodd CM, Bergmann EM, Sheehan JK, Bishop PN. Crystal structure of the biglycan dimer and evidence that dimerization is essential for folding and stability of class I small leucine-rich repeat proteoglycans. J. Biol Chem. 281:13324-13332 (2006); and Fisher, L.W., Termine, J.D., and Young, M.F.: Deduced protein sequence of bone small proteoglycan I (biglycan) shows homology With proteoglycan II (decorin) and several nonconnective tissue proteins in a variety of species. J. Biol. Chem. 264, 4571-4576 (1989)]. The number of the experiments (n) in case of decorin (Figure 9) were as follows: n=7 in the hypoxic group, n=5 in all decorin groups, respectively.

### Results:

Both biglycan (Fig 7) and biglycan core protein (Fig 8) concentration-dependently protected myocytes from hypoxia/reoxygenation induced cell death. Decorin also exerted cytoprotection, but of less degree when compared to biglycan (Fig 9).

Here we would mention that in case of biglycan and biglycan core protein the concentration dependence of cytoprotection seems to show U-shaped curves where the maximum effects are around 30 nm, e.g. in the interval of 20 to 40 nm. (The exact concentration-dependence needs further experimental verification.)

Conclusions: This is the first demonstration that biglycan and its analogue decorin exert cytoprotective effect in hypoxia/ischemia/reoxygenation injury.

### Example 5

### Screening method for "biglycan mimetics"

This example shows the basis of an antibody array assay which can be applied to test molecules mimicking the effect of biglycan in any target organ tissue in vivo or ex vivo.

Method: Tissue samples after treatment with any test substance (small molecules, oligopeptides, polipeptides, etc.) are subjected to a protein array assay for i-NOS, n-NOS, e-NOS, Synaptotagmin, Mcl-1, and Pyk2 as shown in example 1. If test substances change the expression pattern of these proteins, and the changes statistically do not differ from that found in example 1, the test molecules can be considered effective biglycan mimetics.

Conclusions: This is the first assay system to screen for effective biglycan mimetics.

## Claims

1. Use of biglycan or an enhancer of biglycan activity as an active ingredient in the preparation of pharmaceutical compositions for preventing the formation of atherosclerotic plaque.

2. The use according to claim 1, wherein the active ingredient is selected from the following group: biglycans, preferably decorin, inducers or activators of biglycan, delivery systems containing a nucleic acid encoding biglycan and nucleic acids encoding biglycan (naked nucleic acid).

3. The use according to claim 1, wherein the active ingredient is an activator or inducer of the overexpression of endogenous (native) biglycan.

4. Biglycan or an enhancer of biglycan activity for use as an active ingredient in the prevention of the formation of atherosclerotic plaque..

5. The product for use according to claim 4 wherein the active ingredient is selected from the following group: biglycans, preferably decorin, inducers or activators of biglycan, delivery systems containing a nucleic acid encoding biglycan and nucleic acids encoding biglycan (naked nucleic acid).

6. The product for use according to claim 5 wherein the active ingredient is an activator or inducer of the overexpression of endogenous (native) biglycan.

7. The product for use according to claim 6, wherein the activator or inducer of the overexpression of endogenous (native) biglycan is selected from the group of small molecules, preferably by oligo- or polipeptides or Na-salicylate or oxysterol or lysolipids.

8. The product for use according to claim 5, wherein the active ingredient is a gene delivery system containing a nucleic acid encoding biglycan, where said gene delivery system ensures recombinant overexpression of biglycan.

9. The product for use according to any of claims 4 to 8, wherein the active ingredient is incorporated into autografts *ex vivo* before implantation in the case of a bypass surgery, preferably coronary bypass surgery.

## Patentansprüche

1. Verwendung von Biglycan oder eines Verstärkers der Aktivität von Biglycan als Wirkstoff in der Herstellung von pharmazeutischen Zusammensetzungen zur Prävention von atherosklerotischer Plaquebildung.

2. Verwendung gemäß Anspruch 1, wobei der Wirkstoff aus folgender Gruppe ausgewählt ist: Biglycane, vorzugsweise Decorin, Auslöser oder Aktivatoren von Biglycan, Applikationssysteme enthaltend eine für Biglycan kodierende Nukleinsäure und Nukleinsäuren, die für Byglycan kodieren (nackte Nukleinsäure).

3. Verwendung gemäß Anspruch 1, wobei der Wirkstoff ein Aktivator oder Auslöser der Überexpression von endogenem (arteigenem) Biglycan ist.

4. Biglycan oder ein Verstärker der Aktivität von Biglycan zur Verwendung als Wirkstoff zur Prävention von atherosklerotischer Plaquebildung.

5. Produkt zur Verwendung gemäß Anspruch 4, wobei der Wirkstoff aus folgender Gruppe ausgewählt ist: Biglycane, vorzugsweise Decorin, Auslöser oder Aktivatoren von Biglycan, Zuführungssysteme enthaltend eine für Biglycan kodierende Nukleinsäure und Nukleinsäuren, die für Byglycan kodieren (nackte Nukleinsäure).

6. Produkt zur Verwendung gemäß Anspruch 5, wobei der Wirkstoff ein Aktivator oder Auslöser der Überexpression von endogenem (arteigenem) Biglycan ist.

7. Produkt zur Verwendung gemäß Anspruch 6, wobei der Aktivator oder Auslöser der Überexpression von endogenem (arteigenem) Biglycan ausgewählt ist aus der Gruppe kleiner Moleküle, vorzugsweise von Oligo- oder Polypeptiden oder Na-Salicylat oder Oxysterol oder Lysolipiden.

8. Produkt zur Verwendung gemäß Anspruch 5, wobei der Wirkstoff ein Gen-Applikationssystem ist, welches eine für Biglycan kodierende Nukleinsäure enthält und welches die rekombinante Überexpression von Byglycan sicherstellt.

9. Produkt zur Verwendung gemäß einem der Ansprüche 4 bis 8, wobei der Wirkstoff im Falle einer Bypassoperation, vorzugsweise einer koronaren Bypassoperation, vor der Implantation *ex vivo* in Autotransplantante inkorporiert wird.

## Revendications

1. Utilisation de biglycane ou une substance pour l'augmentation de l'activité de biglycane en tant qu'ingrédient actif dans la préparation de compositions pharmaceutiques pour la prévention ou le traitement de plaque athérosclérotique.

2. Utilisation selon la revendication 1, dans lequel l'ingrédient actif est selectionné du groupe suivant: des biglycanes, de préférence décorine, des inducteurs ou des activateurs de biglycane, des systémes de distribution contenant un acide nucléique codant de la biglycane et des acides nucléiques codant de la biglycane (acide nucléique nue).

3. L'utilisation selon la revendication 1, dans lequel l'ingrédient actif est un activateur ou un inducteur de la surexpression de la biglycane endogène (native).

4. Biglycane ou une substance pour l'augmentation de l'activité de biglycane en tant qu'ingrédient actif dans la prévention de la formation de plaque athérosclerotique.

5. Produit pour l'utilisation selon la revendication 4, dans lequel l'ingrédient actif est selectionné du groupe suivant: des biglycanes, de préférence décorine, des inducteurs ou des activateurs de biglycane, des systémes de distribution contenant un acide nucléique codant de la biglycane et des acides nucléiques codant de la biglycane (acide nucléique nue).

6. Produit pour l'utilisation selon la revendication 5, dans lequel l'ingrédient actif est un activateur ou un inducteur de la surexpression de la biglycane endogène (native).

7. Produit pour l'utilisation selon la revendication 6, dans lequel l'activateur ou inducteur de la surexpression de la biglycane endogéne (native) est seléctionné du groupe des petites molécules, de préference par des oligo- ou polypeptides ou de la Na-salicylate ou de l'oxystérole ou par des lysolipides.

8. Produit pour l'utilisation selon la revendication 5, dans lequel l'ingrédient actif est un systéme de distribution des genes contenant une acide nucléique codant de la biglycane, dans lequel ledit système de distribution assure la surexpression récombinante de biglycane.

9. Produit pour l'utilisation selon l'une des revendications 4 à 8, dans lequel l'ingrédient actif est incorporé dans des auto-greffes ex vivo avant l'implantation dans le cas d'une chirurgie de pontage, de préfernce une chrirurgie de pontage coronaire.
